# EUROPÄISCHE PATENTANMELDUNG

(11) **EP 0 968 707 A2**
(43) Veröffentlichungstag der Anmeldung: **05.01.2000**
(21) Anmeldenummer: 99111704.5
(22) Anmeldetag: 17.06.1999
(51) Int. Cl.: A61K 7/48, A61K 38/48

(54) **Gegen Akne und entzündete Comedonen wirksame Zubereitungen enthaltend Serin-Proteasen und ein oder mehrere Calciumsalze**

(30) Priorität: 03.07.1998 DE 19829789
(71) Anmelder: Beiersdorf Aktiengesellschaft, 20245 Hamburg (DE)
(72) Erfinder: Schönrock, Uwe, Dr., 23866 Nahe (DE); Christiansen, Michael, 25436 Tornesch (DE); Untiedt, Sven, Dr., 20259 Hamburg (DE); Steinke, Sigrid, 21075 Hamburg (DE); Herpens, Andreas, 21465 Reinbek (DE); Max, Heiner, Dr., 22529 Hamburg (DE)

(57) **Zusammenfassung**

Kosmetische oder dermatologische Zubereitungen mit einem Gehalt an einer oder mehreren Serin-Proteasen und einem oder mehreren Calciumsalzen bzw. die Verwendung von Kombinationen aus einer oder mehreren Serin-Proteasen und einem oder mehreren Calciumsalzen gegen das Erscheinungsbild der unreinen Haut bzw. der Akne.

## Beschreibung

Die vorliegende Erfindung betrifft Wirkstoffe und Zubereitungen, solche Wirkstoffe enthaltend, welche gegen unreine Haut bzw. Akne wirksam sind.

Bei der unreinen Haut sind neben anderen Einflüssen bakterielle Sekundärinfektionen von ätiologischer Bedeutung. Einer der wichtigsten Mikroorganismen, der in Zusammenhang mit unreiner Haut steht, ist Propionibacterium acnes.

Unreine Haut und/oder Komedonen beeinträchtigen das Wohlbefinden der Betroffenen aber selbst in leichten Fällen. Da praktisch jeder oder jede Jugendliche von unreiner Haut irgendeiner Ausprägung betroffen ist, besteht bei vielen Personen Bedarf, diesem Zustande abzuhelfen.

Akne ist eine Hauterkrankung mit verschiedenen Formen und Ursachen, gekennzeichnet durch nicht entzündliche und entzündliche Knötchen, ausgehend von verstopften Haarfollikeln (Komedonen), die zur Pustel-, Abszeß- und Narbenbildung führen kann. Am häufigsten ist die Acne vulgaris, die vorwiegend in der Pubertät auftritt. Ursächliche Bedingungen für die Acne vulgaris sind die Verhornung und Verstopfung der Haarfollikel-Mündung, die vom Blutspiegel der männlichen Sexualhormone abhängige Talgproduktion und die Produktion freier Fettsäuren und gewebeschädigender Enzyme durch Bakterien (*Propionibacterium acnes*).

Beim Vollbilde der Akne, aber auch bei leichteren Ausprägungen, sind Entzündungen der Aknepusteln häufige Folge. Der Stand der Technik ließ es an Wirkstoffen, die eine befriedigende Behandlung im Sinne einer Heilung mangeln.

Aufgabe der vorliegenden Erfindung war es also, einen gegen unreine Haut bzw. Akne wirksamen Stoff zu finden.

Es hat sich überraschenderweise gezeigt, und darin liegt die Lösung der Aufgabe, daß kosmetische oder dermatologische Zubereitungen mit einem Gehalt an einer oder mehreren Serin-Proteasen und einem oder mehreren Calciumsalzen bzw. die Verwendung von Kombinationen aus einer oder mehreren Serin-Proteasen und einem oder mehreren Calciumsalzen gegen das Erscheinungsbild der unreinen Haut bzw. der Akne den Nachteilen des Standes der Technik abhilft.

Ganz erstaunlich war eine deutliche Erhöhung der Wirksamkeit der erfindungsgemäß eingesetzten Wirkstoffkombinationen bzw. Zubereitungen gemäß der Erfindung gegen unreine Haut bzw. Akne im Vergleich zu Zubereitungen ohne Zusatz von Calciumionen.

Ferner war erstaunlich, daß sich die erfindungsgemäß eingesetzten Wirkstoffkombinationen bzw. Zubereitungen gemäß der Erfindung durch signifikant verstärkte Aktivität gegenüber Zubereitungen ohne Zusatz von Calciumionen auszeichnet.

Zwar wird in der Schrift WO 93/19731 die Verwendung von Proteasen gegen - unter anderem - Akne beschrieben, die a.a.O. beschriebenen Proteasen legten jedoch nicht die vorteilhaften Eigenschaften der erfindungsgemäß verwendeten Zubereitungen bzw. Verwendung von Wirkstoffkombinationen.

Serin-Proteasen enthalten im aktiven Zentrum einen für die Katalyse essentiellen L-Serin-Rest, welcher Teil der sogenannten katalytischen Triade (aus drei Aminosäuren) ist, die einander in der Tertiärstruktur der Enzyme nahekommen. Ein L-Histidinrest agiert als Akzeptor für das Proton der OH-Gruppe des Serins, welches bei der Katalyse das Protein nucleophil angreift. Ein L-Aspartatrest wirkt stabilisierend auf den Übergangszustand.

Erfindungsgemäß günstig zu verwendende Serin-Proteasen sind beispielsweise Subtilisin-Proteasen, insbesondere eine Protease die von der Gesellschaft Novo Nordisk A/S, Bagsværd, Dänemark, unter der Bezeichnung 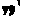 SP 544 Protease" erhältlich ist.

Das oder die Calciumsalze können vorteilhaft gewählt werden aus der Gruppe der leicht und mäßig wasserlöslichen Calciumsalze, insbesonde die Calciumsalze mit folgenden Anionen: Chlorid, organische Anionen wie z.B. Lactat, Acetat, Benzoat, Propionat, Tartrat, Citrat, ferner Aminosäuresalze des Calciums, Calcium-Salze von Schieferöl-Sulfonsäuren und andere mehr.

Die erfindungsgemäß verwendeten kosmetischen oder dermatologischen Zubereitungen enthalten bevorzugt 0,0001 - 5,00 Gew.-% einer oder mehrerer Serin-Proteasen, insbesondere Subtilisin-Proteasen, bezogen auf das Gesamtgewicht der Zubereitungen.

Die erfindungsgemäß verwendeten kosmetischen oder dermatologischen Zubereitungen enthalten bevorzugt 0,0001 - 5,00 Gew.-% an Calciumionen, bezogen auf das Gesamtgewicht der Zubereitungen.

Erfindungsgemäß günstig, aber dennoch fakultativ können Antioxidantien in Kombination mit Serin-Proteasen verwendet werden, wobei alle für kosmetische und/oder dermatologische Anwendungen geeigneten oder gebräuchlichen Antioxidantien erfindungsgemäß vorteilhaft in Betracht kommen. Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, ψ-Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Seien und dessen Derivate (z.B. Seienmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist vorteilhaft, den erfindungsgemäß verwendeten Zubereitungen weitere gegen Akne wirksame Substanzen oder antientzündliche Wirkstoffe zuzugeben, beispielsweise Batylalkohol (α-Octadecylglycerylether), Selachylalkohol (α-9-Octadecenylglycerylether), Chimylalkohol (α-Hexadecylglycerylether) und/oder Bisabolol.

Es ist dem Fachmanne natürlich bekannt, daß anspruchsvolle kosmetische Zusammensetzungen zumeist nicht ohne die üblichen Hilfs und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Antioxidantien, Insektenrepellentien, Alkohol, Wasser, Salze, antimikrobiell, proteolytisch oder keratolytisch wirksame Substanzen usw.

Es ist von Vorteil, den Wassergehalt der Formulierungen geringer als 65 Gew.-% und den Alkoholgehalt geringer als 20 Gew.-% zu halten. Ferner hat es sich als bevorzugt herausgestellt, den Gehalt an Chelatbildnern so gering wie möglich zu halten, typischerweise geringer als 0,5 Gew.-%, insbesondere geringer als 0,01 Gew.-%, bezogen auf das Gesamtgewicht der Zubereitungen möglichst ganz auf Chelatbildner zu verzichten.

Ferner ist von Vorteil, auf die Gegenwart von Glycosidasen zu verzichten.

Es ist besonders vorteilhaft, den erfindungsgemäß verwendeten Zubereitungen Puffersubstanzen zuzufügen. Insbesondere vorteilhaft ist, wenn die Zubereitungen auf pH-Werte von 6,5 oder kleiner, insbesondere 6,0, abgepuffert werden.

Zur Anwendung werden die erfindungsgemäßen kosmetischen und dermatologischen Zubereitungen in der für Kosmetika üblichen Weise auf die Haut in ausreichender Menge aufgebracht.

Erfindungsgemäß verwendete kosmetische und dermatologische Zubereitungen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, einen festen Stift oder auch ein Aerosol darstellen.

Es ist auch möglich und vorteilhaft im Sinne der vorliegenden Erfindung, die erfindungsgemäß verwendeten Serin-Proteasen in wäßrige Systeme bzw. Tensidzubereitungen zur Reinigung der Haut einzufügen.

Die erfindungsgemäß verwendeten kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Die Lipidphase kann vorteilhaft gewählt werden aus folgender Substanzgruppe:
- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäß verwendete feste Stifte enthalten z.B. natürliche oder synthetische Wachse, Fettalkohole oder Fettsäureester.

Es ist vorteilhaft, den erfindungsgemäß verwendeten Zubereitungen weitere gegen Akne wirksame Substanzen zuzugeben, beispielsweise Substanzen, die gegen *Propionibacterium acnes* wirksam sind (etwa solche, die in DE-OS 42 29 707, DE-OS 43 05 069, DE-OS 43 07 976, DE-OS 43 37 711, DE-OS 43 29 379 beschrieben werden) aber auch andere gegen Akne wirksame Substanzen, beispielsweise all-trans-Retinsäure, 13-cis-Retinsäure und verwandte Stoffe) oder antientzündliche Wirkstoffe, beispielsweise Batylalkohol (α-Octadecylglycerylether), Selachylalkohol (α-9-Octadecenylglycerylether), Chimylalkohol (α-Hexadecylglycerylether) und/oder Bisabolol sowie Antibiotika und/oder Keratolytika.

Alle gängigen gegen Akne wirksamen Substanzen können vorteilhaft genutzt werden, insbesondere Benzoylperoxid, Bituminosulfonate (Ammonium-, Natriumsalze von Schieferöl-Sulfonsäuren), Salicylsäure (2-Hydroxybenzoesäure), Miconazol (1-[2-(2,4-Dichlorbenzyloxy)-2-(2,4-dichlorphenyl)-ethyl]-imidazol) und Derivate, Adapalen (6-[3-(1-Adamantyl)-4-methoxyphenyl]-2-naphthoäsäure), Azelainsäure (Nonandisäure), Mesulfen (2,7-Dimethylthianthren, C₁₄H₁₂S₂) sowie Aluminiumoxid, Zinkoxid und/oder feinverteilter Schwefel.

Die Menge der zusätzlich eingesetzten Antiaknemittel (eine oder mehrere Verbindungen) in den Zubereitungen beträgt vorzugsweise 0,01 bis 30 Gew.-%, besonders bevorzugt 0,1 - 20 Gew.-%, insbesondere 1 - 10 Gew.-%, bezogen auf das Gesamtgewicht der Zubereitung.

Bevorzugt können die Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische und/oder dermatologische Zubereitungen zur Verfügung zu stellen, die die Haut vor dem gesamten Bereich der ultravioletten Strahlung schützen.

Enthalten die erfindungsgemäß verwendeten Zubereitungen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, z.B. 3-(4-Methylbenzyliden)campher, 3-Benzylidencampher;
- 4-Aminobenzoësäure-Derivate, z.B. 4-(Dimethylamino)-benzoësäure(2-ethylhexyl)ester, 4-(Dimethylamino)benzoësäureamylester;
- Ester der Zimtsäure, z.B. 4-Methoxyzimtsäure(2-ethylhexyl)ester, 4-Methoxyzimtsäureisopentylester;
- Ester der Salicylsäure, z.B. Salicylsäure(2-ethylhexyl)ester, Salicylsäure(4-isopropylbenzyl)ester, Salicylsäurehomomenthylester,
- Derivate des Benzophenons, z.B. 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, z.B. 4-Methoxybenzalmalonsäuredi(2-ethylhexyl)-ester,
- 2,4,6-Trianilino-(p-carbo-2'-ethyl-1'-hexyloxy) -1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, wie z.B. 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Enthalten die erfindungsgemäß verwendeten Zubereitungen UVA-Filtersubstanzen, können diese erfindungsgemäß vorteilhaft gewählt werden aus der Gruppe der Derivate des Dibenzoylmethans, z.B. 1-(4'-tert.Butylphenyl)-3-(4'-methoxyphenyl)propan-1,3-dion und 1-Phenyl-3-(4'-isopropylphenyl)propan-1,3-dion.

Die erfindungsgemäß verwendeten Zubereitungen können auch weitere anorganische Pigmente enthalten, die üblicherweise in der Kosmetik zum Schutze der Haut vor UV-Strahlen verwendet werden. Dabei handelt es sich um Oxide des Titans, Eisens, Zirkoniums, Siliciums, Mangans, Aluminiums, Cers und Mischungen bzw. Abwandlungen davon. Besonders bevorzugt handelt es sich um Pigmente auf der Basis von Titandioxid.

Insbesondere werden Gemische der vorstehend genannten Lösemittel verwendet. Bei alkoholischen Lösemitteln kann Wasser ein weiterer Bestandteil sein.

Es ist von Vorteil, den Tensidgehalt der Formulierungen möglichst gering zu halten, insbesondere geringer als 5 Gew.-%.

Ansonsten gelten für diese Gruppe an kosmetischen und/oder dermatologischen Zubereitungen die üblichen Anforderungen, die der Fachmann an solche Zubereitungen und deren Inhaltsstoffe stellt.

Die folgenden Beispiele wollen die vorliegende Erfindung erläutern, ohne daß eine Beschränkung auf den Gehalt der Beispiele beabsichtigt ist. Die Mengenangaben bedeuten stets, sofern nichts Anderes angegeben ist, Gewichts-%, bezogen auf das Gesamtgewicht der jeweiligen Zubereitungen.

### Beispiel 1

| Akne-Tonic | |
|---|---|
| | Gew.-% |
| Poloxamer 124 | 10,00 |
| Butylenglycol | 20,00 |
| PEG-40-hydriertes Rizinusöl | 2,00 |
| Retinylpalmitat | 0,36 |
| Protease SP 544 | 0,06 |
| CaCl₂ | 0,50 |
| Ethanol | 10,00 |
| Wasser | ad 100,00 |

### Beispiel 2

| Akne-Tonic | |
|---|---|
| | Gew.-% |
| Poloxamer 124 | 10,00 |
| Butylenglycol | 20,00 |
| Polysorbat 20 | 2,00 |
| Protease SP 544 | 0,06 |
| CaCl₂ | 0,50 |
| Ethanol | 10,00 |
| Wasser | ad 100,00 |

### Beispiel 3

| O/W Lotion | |
|---|---|
| | Gew.-% |
| Paraffinöl (DAB 9) | 8,00 |
| Isopropylpalmitat | 3,00 |
| Petrolatum | 4,00, |
| Cetylstearylalkohol | 2,00 |
| PEG 40 Rizinusöl | 0,50 |
| Natriumcetylstearylsulfat | 0,50 |
| Natrium Carbomer | 0,40 |
| Glycerin | 3,00 |
| α-Tocopherol | 0,20 |
| Octylmethoxycinnamat | 5,00 |
| Butylmethoxydibenzoylmethan | 1,00 |
| Konservierungsmittel, Farbstoffe, Parfüm | q.s. |
| Protease SP 544 | 0,06 |
| Ca-Acetat | 0,50 |
| Wasser | ad 100,00 |

### Beispiel 4

| O/W Creme | |
|---|---|
| | Gew.-% |
| Paraffinöl (DAB 9) | 7,00 |
| Avocadoöl | 4,00 |
| Glycerylmonostearat | 2,00 |
| Natriumlactat | 3,00 |
| Glycerin | 3,00 |
| Konservierungsmittel, Farbstoffe, Parfüm | q.s. |
| Protease SP 544 | 0,06 |
| Ca-Lactat | 0,50 |
| Wasser | ad 100,00 |

### Beispiel 5

| Liposomenhaltiges Gel | |
|---|---|
| | Gew.-% |
| Lecithin | 6,00 |
| Schibutter | 3,00 |
| α-Tocopherol | 0,20 |
| Protease SP 544 | 0,06 |
| CaCl₂ | 0,50 |
| Natrium PCA | 0,50 |
| Xanthan Gummi | 1,40 |
| Sorbitol | 3,00 |
| Konservierungsmittel, Farbstoffe, Parfüm | q.s. |
| Wasser | ad 100,00 |

## Patentansprüche

1. Kosmetische oder dermatologische Zubereitungen mit einem Gehalt an einer oder mehreren Serin-Proteasen und einem oder mehreren Calciumsalzen.

2. Verwendung von Kombinationen aus einer oder mehreren Serin-Proteasen und einem oder mehreren Calciumsalzen gegen das Erscheinungsbild der unreinen Haut bzw. der Akne.

3. Zubereitungen nach Anspruch 1 bzw. Verwendung nach Anspruch 2, dadurch gekennzeichnet, daß die Serin-Protease oder die Serin-Proteasen in kosmetischen oder dermatologischen Zubereitungen in Konzentrationen von 0,0001 - 5,000 Gew.-% vorliegt, bezogen auf das Gesamtgewicht der Zubereitungen.

4. Zubereitungen nach Anspruch 1 bzw. Verwendung nach Anspruch 2, dadurch gekennzeichnet, daß in den kosmetischen oder dermatologischen Zubereitungen Calciumionen in Konzentrationen von 0,0001 - 5,000 Gew.-% vorliegen, bezogen auf das Gesamtgewicht der Zubereitungen.

5. Verwendung nach Anspruch 2, dadurch gekennzeichnet, daß den Zubereitungen weitere antientzündliche Wirkstoffe zuzugeben werden, gewählt aus der Gruppe der Antioxidantien, ferner Batylalkohol, Selachylalkohol, Chimylalkohol und Bisabolol.
